# EUROPEAN PATENT APPLICATION

(11) **EP 4 623 898 A1**
(43) Date of publication of application: **01.10.2025**
(21) Application number: 24209547.9
(22) Date of filing: 29.10.2024
(51) Int. Cl.: A61K 9/00, A61K 9/20

(54) **LISDEXAMFETAMINE DIMESYLATE ORODISPERSIBLE TABLET AND PROCESS FOR PREPARATION THEREOF**

(30) Priority: 28.03.2024 IN 202411025463
(71) Applicant: Athena Pharmaceutiques SAS, 78430 Louveciennes (FR)
(72) Inventor: CHAUDHARI, Mahendra Baliram, 400602 Maharashtra (IN); CHANDWANI, Omprakash Doulatram, 421301 Maharashtra (IN); NEHETE, Nitin Pandharinath, 421201 Maharashtra (IN); CHAUDHARI, Amol Yuvraj, 421202 Maharashtra (IN); SHAHANE, Anita Kunal, 421301 Maharashtra (IN)
(74) Representative: Grünecker Patent- und Rechtsanwälte PartG mbB

(57) **Abstract**

The present disclosure relates to a lisdexamfetamine dimesylate orodispersible tablet and a process for preparation thereof. The lisdexamfetamine dimesylate orodispersible tablet comprises: co-sifting lisdexamfetamine dimesylate; a binder and a diluent; dry mixing and blending the sifted lisdexamfetamine dimesylate, the binder and the diluent to form a blended dry mix; co-sifting and blending the diluent, a cushioning agent, a disintegrating agent, a sweetener, and an antistatic agent to prepare an intermediate blend; blending the blended dry mix and the intermediate blend; lubricating the blended dry mix and the intermediate blend to form ready to compress drug granules; and compressing the ready to compress drug granules to form compressed lisdexamfetamine dimesylate orodispersible tablet.

## Description

### FIELD OF THE PRESENT DISCLOSURE

The present disclosure generally relates to an orally disintegrating tablet. In particular, the present disclosure relates to a lisdexamfetamine dimesylate orodispersible tablet and process for preparation thereof.

### BACKGROUND

Lisdexamfetamine dimesylate, the dimesylate salt of L-Lysyl-d-amfetamine with molecular formula C₁₇H₃₃N₃O₇S₂ is also known by a chemical name "(2S)-2,6-diamino-N-[(1S)-1-methyl-2-phenylethyl]-hexanamide, dimethanesulfonate". IUPAC name of the Lisdexamphetamine dimesylate is (2S)-2,6-diamino-N-[(2S)-1-phenylpropan-2-yl] hexanamide; methanesulfonic acid.

Chemical structure of Lisdexamfetamine dimesylate is represented below:

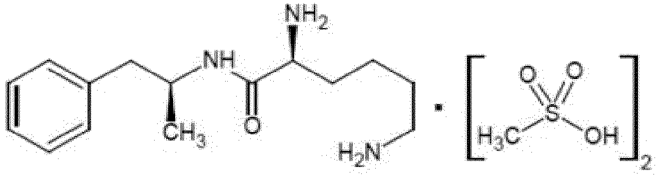

Lisdexamfetamine dimesylate generally indicates the treatment of Attention Deficit Hyperactivity Disorder (ADHD) and moderate to severe Binge Eating Disorder (BED) in adults.

Tablets and capsules have drawbacks in that water is needed when they are taken, and they are not well-accepted by aged people and those having difficulty in swallowing. It has been reported that Dysphagia (difficulty in swallowing) is common among all age groups and more specifically with geriatric population along with institutionalized patients and patients with nausea, vomiting and motion sickness complications.

During the last decade there is a need for an upcoming generation of pharmaceutical products and medicaments which can be taken anywhere. The need further includes a suitable type of formulations under the form of orodispersible form or are rapidly dissolved and have the characteristics of dissolving or melting or disintegrating in the oral cavity in only a few seconds in absence of water.

These formulations are quickly disintegrable or soluble when put in the oral cavity, and thus are suitable for the aged people, and those having difficulty in swallowing. Moreover, since the formulation disintegrates inside the mouth, the drug may be absorbed in the oral, pharyngeal, and gastrointestinal regions.

Lisdexamfetamine dimesylate is highly deliquescent material by its nature, which on being exposed to the moisture by any means absorbs the moisture and does not remain in solid form.

Lisdexamfetamine dimesylate is present in the market in the form of capsules, and chewable tablets. Multitude of patient's group such as geriatrics, patients with mental retardness, uncooperative patients, patients with nausea, and patients with less liquid intake or diet etc., face difficulty in swallowing lisdexamfetamine dimesylate in the available dosage forms.

Accordingly, there is a need for lisdexamfetamine dimesylate orodispersible composition that addresses the deliquescence problem of lisdexamfetamine dimesylate. It is therefore highly desirable to develop a lisdexamfetamine dimesylate orodispersible composition which is orally disintegrating that permits rapid release of the drug from the granules and allows rapid absorption in the body after administration. Further it is desirable to develop a lisdexamfetamine dimesylate orodispersible tablet that is stable, palatable, and disintegrates fast. Moreover, there is a need for manufacturing process in which the process time for preparing lisdexamfetamine dimesylate orodispersible composition is low, and the production cost is low. Furthermore, there is a need of manufacturing process with controlled humidity to minimize exposure of the deliquescent material to moisture during the production process.

### SUMMARY

To achieve the foregoing and other objects and needs, the present disclosure provides a lisdexamfetamine dimesylate orodispersible composition which is in the form of lisdexamfetamine dimesylate orodispersible tablet and process for preparation thereof. The lisdexamfetamine dimesylate orodispersible tablet developed by the inventor is an orally disintegrating tablet that permits rapid release of the drug from the granules and allows rapid absorption in the body after administration. Further, the present disclosure provides a lisdexamfetamine dimesylate orodispersible tablet that is stable, palatable, and disintegrates fast. The present disclosure further provides a manufacturing process in which the process time for preparing lisdexamfetamine dimesylate orodispersible composition is low, and the production cost is low. Furthermore, the present disclosure further provides a manufacturing process with controlled humidity to minimize exposure of the deliquescent material to moisture during the production process.

The present disclosure provides a synergistic combination of the API with the excipients in specific ratio to address the deliquescent nature of lisdexamfetamine dimesylate. The synergistic combination enhances the disintegration time of the orodispersible tablet. The present disclosure further imparts improved palatability to the tablet formulation of lisdexamfetamine dimesylate. The present disclosure provides a humidity-controlled manufacturing process wherein the tablet formulation is manufactured in an environment with controlled humidity to minimize exposure of the deliquescent material to moisture during the production process. Particularly, the manufacturing process employs direct compression techniques during the manufacturing process in controlled humidity level of NMT 40 % to reduce exposure to moisture.

The present disclosure provides a lisdexamfetamine dimesylate composition of orally disintegrating tablet which overcomes the drawbacks of capsules and chewable tablets. The synergistic combination of the API with the excipients in specific ratio is formulated to address the deliquescent nature of lisdexamfetamine dimesylate and provide a stable lisdexamfetamine dimesylate composition. The dissolution of the lisdexamfetamine orodispersible tablet composition is more than 85% in 15 minutes.

In an embodiment, the present disclosure relates to a lisdexamfetamine dimesylate orodispersible tablet. The lisdexamfetamine dimesylate orodispersible tablet comprises about 8 to about 12 percent by weight of lisdexamfetamine dimesylate; about 45 to about 55 percent by weight of a diluent; about 10 to about 20 percent by weight of a binder; about 8 to about 12 percent by weight of a cushioning agent; about 6 to about 10 percent by weight of a disintegrating agent; about 1 to about 4 percent by weight of a sweetener; about 1 to about 2 percent by weight of an antistatic agent; and about 1 to about 2 percent by weight of a lubricant.

In one or more embodiments, the dosage amount of the lisdexamfetamine dimesylate orodispersible tablet is 10 mg per tablet; 20 mg per tablet; 30 mg per tablet; 40 mg per tablet; 50 mg per tablet; 60 mg per tablet and 70 mg per tablet.

In one or more embodiments, the diluent is mannitol granular 200SD, and the binder is partially pregelatinized maize starch.

In one or more embodiments, the cushioning agent is microcrystalline cellulose, the disintegrating agent is crospovidone, the sweetener is sucralose, the antistatic agent is colloidal anhydrous silica, and the lubricant is magnesium stearate.

In one or more embodiments, the particle size for a drug granule forming the lisdexamfetamine dimesylate orodispersible tablet is not more than 400 microns.

In one or more embodiments, the friability is less than 2%, the disintegration time is less than 3 minutes at 37°C ±2°C (without disc), and the hardness is from about 30 N to about 70 N.

In one or more embodiments, the dissolution is more than 85% in 15 minutes.

In one or more embodiments, the particle size of the lisdexamfetamine dimesylate is not more than 100 microns.

In another embodiment, the present disclosure relates to a process for preparation of a lisdexamfetamine dimesylate orodispersible tablet. The process comprises co-sifting lisdexamfetamine dimesylate, a binder, and a diluent; dry mixing and blending the sifted lisdexamfetamine dimesylate, the binder, and the diluent to form a blended dry mix; co-sifting and blending the diluent, a cushioning agent, a disintegrating agent, a sweetener, and an antistatic agent to prepare an intermediate blend; blending the blended dry mix and the intermediate blend; lubricating the blended dry mix and the intermediate blend to form ready to compress drug granules; and compressing the ready to compress drug granules to form compressed lisdexamfetamine dimesylate orodispersible tablet.

In one or more embodiments, the dosage amount of the lisdexamfetamine dimesylate orodispersible tablet is 10 mg per tablet; 20 mg per tablet; 30 mg per tablet; 40 mg per tablet; 50 mg per tablet; 60 mg per tablet and 70 mg per tablet.

In one or more embodiments, the diluent is mannitol granular 200SD, and the binder is partially pregelatinized maize starch.

In one or more embodiments, the cushioning agent is microcrystalline cellulose; the disintegrating agent is crospovidone, the sweetener is sucralose, the antistatic agent is colloidal anhydrous silica, and the lubricant is magnesium stearate.

In one or more embodiments, the friability is less than 2%, disintegration time is less than 3 minutes at 37°C ±2°C (without disc), and the hardness is from about 30 N to about 70 N.

In one or more embodiments, the step of compressing employs direct compression technique in controlled humidity level of NMT 40 % to reduce exposure to moisture.

In an embodiment, further comprising packaging of compressed lisdexamfetamine dimesylate orodispersible tablets in moisture resistant ALU peel off blister package.

In another embodiment, further comprising packaging of compressed lisdexamfetamine dimesylate orodispersible tablets in moisture resistant ALU-ALU package.

### BRIEF DESCRIPTION OF THE FIGURES

The advantages and features of the present disclosure will become better understood with reference to the following detailed description and claims taken in conjunction with the accompanying drawing, in which:
FIG. 1 illustrates a process flow for preparation of a lisdexamfetamine dimesylate orodispersible tablet, in accordance with an exemplary embodiment of the present disclosure.
FIG. 2 illustrates process details of dry mixing of lisdexamfetamine dimesylate, binder and diluent, in accordance with an exemplary embodiment of the present disclosure.
FIG. 3 illustrates process details of preparation of lubricated blend/ready to compress drug granules, in accordance with an exemplary embodiment of the present disclosure.
FIG. 4 illustrates details of preparation of compressed lisdexamfetamine dimesylate orodispersible tablets, in accordance with an exemplary embodiment of the present disclosure.

### DETAILED DESCRIPTION

The exemplary embodiments described herein detail for illustrative purposes are subject to many variations in structure and design. It should be emphasized, however, that the present disclosure is not limited to the lisdexamfetamine dimesylate orodispersible tablet and process for preparation thereof, as shown and described. It is understood that various omissions and substitutions of equivalents are contemplated as circumstances may suggest or render expedient, but these are intended to cover the application or implementation without departing from the spirit or scope of the claims of the present disclosure. Also, it is to be understood that the phraseology and terminology used herein is for the purpose of description and should not be regarded as limiting.

The use of terms "including," "comprising," or "having" and variations thereof herein is meant to encompass the items listed thereafter and equivalents thereof as well as additional items.

Further, the terms, "a" and "an" herein do not denote a limitation of quantity, but rather denote the presence of at least one of the referenced items.

As used herein, the term "about" will be understood by persons of ordinary skill in the art and will vary to some extent depending upon the context in which it is used. If there are uses of the term which are not clear to persons of ordinary skill in the art, given the context in which it is used, "about" will mean up to plus or minus 10 % of the particular term.

The present disclosure provides a lisdexamfetamine dimesylate orodispersible tablet and process for preparation thereof, that is an oral dosage form with orally disintegrating properties which eliminates the requirement of consuming lisdexamfetamine dimesylate with help of water and disintegrates rapidly in the saliva. The lisdexamfetamine dimesylate orodispersible tablet developed by the inventor is an orally disintegrating tablet that permits rapid release of the drug from the granules and allows rapid absorption in the body after administration. Further, the lisdexamfetamine dimesylate orodispersible tablet and process for preparation thereof requires lesser process time for preparation, lesser number of excipients and the production cost is low, and it is stable, palatable, and disintegrates fast.

The present disclosure provides a synergistic combination of the API with the excipients in specific ratio to address the deliquescent nature of lisdexamfetamine dimesylate. The synergistic combination enhances the disintegration time of the orodispersible tablet. The present disclosure further imparts improved palatability to the tablet formulation of lisdexamfetamine dimesylate. The present disclosure provides a humidity-controlled manufacturing process wherein the tablet formulation is manufactured in an environment with controlled humidity to minimize exposure of the deliquescent material to moisture during the production process. Particularly, the manufacturing process employs direct compression techniques during the manufacturing process in controlled humidity level of NMT 40 % to reduce exposure to moisture.

In one embodiment, a lisdexamfetamine dimesylate orodispersible tablet is disclosed. Herein, the "lisdexamfetamine dimesylate orodispersible tablet" and "lisdexamfetamine dimesylate ODT" have been interchangeably used hereinafter without any limitations. The lisdexamfetamine dimesylate orodispersible tablet comprises about 8 to about 12 percent by weight of lisdexamfetamine dimesylate; about 45 to about 55 percent by weight of a diluent; about 10 to about 20 percent by weight of a binder; about 8 to about 12 percent by weight of a cushioning agent; about 6 to about 10 percent by weight of a disintegrating agent; about 1 to about 4 percent by weight of a sweetener; about 1 to about 2 percent by weight of an antistatic agent; and about 1 to about 2 percent by weight of a lubricant.

As used herein, lisdexamfetamine dimesylate is the active substance. Lisdexamfetamine dimesylate, the dimesylate salt of L-Lysyl-d-amfetamine with molecular formula C₁₇H₃₃N₃O₇S₂ is also known by a chemical name "(2S)-2,6-diamino-N-[(1S)-1-methyl-2-phenylethyl]-hexanamide, dimethanesulfonate". IUPAC name of the Lisdexamphetamine dimesylate is (2S)-2,6-diamino-N-[(2S)-1-phenylpropan-2-yl] hexanamide; methanesulfonic acid.

Structural formula of lisdexamfetamine dimesylate shows that the molecule of lisdexamfetamine dimesylate contains two chiral centres. Thus, there are four possible enantiomers of this compound: (2S)-2,6-diamino-N-[(2S)-1-phenylpropan-2-yl]hexanamide; (2R)-2,6-diamino-N-[(2S)-1-phenylpropan-2-yl]hexanamide; (2S)-2,6-diamino-N-[(2R)-1-phenylpropan-2-yl]hexanamide; and (2R)-2,6-diamino-N-[(2R)-1-phenylpropan-2-yl]hexanamide.

Lisdexamfetamine dimesylate is a white or almost white crystalline powder which is therapeutically categorized as a CNS stimulant drug also called amfetamines. Lisdexamfetamine dimesylate is freely soluble in methanol; soluble in ethanol; very slightly soluble in acetone, chloroform and toluene; and practically insoluble in tetrahydrofuran.

Characteristically, lisdexamfetamine dimesylate is hygroscopic in nature and generally indicates the treatment of Attention Deficit Hyperactivity Disorder (ADHD), and moderate to severe binge eating disorder (BED) in adults.

In one or more embodiments, the particle size of the lisdexamfetamine dimesylate is not more than 100 microns.

In one or more embodiments, the dosage amount of the lisdexamfetamine dimesylate orodispersible tablet is 10 mg per tablet; 20 mg per tablet; 30 mg per tablet; 40 mg per tablet; 50 mg per tablet; 60 mg per tablet and 70 mg per tablet.

In one or more embodiments, the diluent is mannitol granular 200SD.

As used herein, the mannitol granular 200SD is used in preparation of the lisdexamfetamine dimesylate orodispersible tablet. Mannitol granular 200SD is used to adjust the average weight of lisdexamfetamine dimesylate orodispersible tablets. Mannitol granular 200SD also enhances the flow properties and mouth feel of lisdexamfetamine dimesylate orodispersible tablet formulation. Mannitol granular 200SD is divided in two parts to enhance the uniformity and distribution of drugs in formulation. In an example, the mannitol granular 200SD is Pearlitol^{®}200SD.

In one or more embodiments, the binder is partially pregelatinized maize starch.

As used herein, the partially pregelatinized maize starch is used in preparation of the lisdexamfetamine dimesylate orodispersible tablet. Lisdexamfetamine dimesylate as an active substance is a deliquescent molecule, by that virtue, on keeping it at room temperature conditions it converts in liquid form. Therefore, in order to keep the active lisdexamfetamine dimesylate in its solid form, swellable partially pregelatinized maize starch is used in this lisdexamfetamine dimesylate orodispersible tablet formulation. Partially pregelatinized maize starch improves the flow properties of powder guaranteeing uniformity. In contact with aqueous dissolution medium the Pregelatinized maize starch swells and triggers the disintegration. It significantly decreases the adhesion of powders. In an example, the partially pregelatinized maize starch is Starch1500^{®}.

In one or more embodiments, the cushioning agent is microcrystalline cellulose.

As used herein, the microcrystalline cellulose is used in preparation of the lisdexamfetamine dimesylate orodispersible tablet. Microcrystalline cellulose is crystalline in nature. Microcrystalline cellulose enhances the flow of powder and helps in tablet compression to give desired hardness. It also acts as a cushioning agent. Due to the plastic nature of microcrystalline cellulose, it is often used as an excipient for both tablet compression and pelletizing processes. Microcrystalline cellulose as a cushioning agent fills the space between the particles and dampens the effect of the compressive force occurring through the tableting process. In an example, microcrystalline cellulose is Vivapur^{®} 112.

In one or more embodiments, the disintegrating agent is crospovidone.

As used herein, crospovidone is used in preparation of the lisdexamfetamine dimesylate orodispersible tablet. Crospovidone is a water insoluble tablet disintegrant and dissolution agent. Crospovidone rapidly exhibits high capillary activity and pronounced hydration capacity with little tendency to form gels. In an example, crospovidone is Kollidone^{®}CL.

In one or more embodiments, the sweetener is sucralose.

As used herein, sucralose is used in preparation of the lisdexamfetamine dimesylate orodispersible tablet. Sucralose is white to off-white colored, free flowing, crystalline powder which has sweetening power approximately 300-1000 times that of sucrose and has no after taste.

In one or more embodiments, the antistatic agent is colloidal anhydrous silica.

As used herein, colloidal anhydrous silica is used in preparation of the lisdexamfetamine dimesylate orodispersible tablet. Colloidal anhydrous silica is an adsorbent, anticaking agent, and glidant. It enhances the flow of powder. In an example, colloidal anhydrous silica is Aerosil^{®} 200.

In one or more embodiments, the lubricant is magnesium stearate.

As used herein, magnesium stearate is used in preparation of the lisdexamfetamine dimesylate orodispersible tablet. Magnesium stearate is an impalpable powder with low bulk density. It is very fine and greasy in texture, light in weight, white in color, precipitated or milled in nature. Functionally, it is used as a lubricant in capsule and tablet manufacturing. Magnesium stearate powder, because of its characteristic greasiness adheres readily to the surface of tablet to ensure tablet is cleanly ejected without cracking or breakage in the pharmaceutical industry.

In one or more embodiments, the particle size for drug granules forming the tablet is not more than 400 microns.

In one or more embodiments, the friability is less than 2%, disintegration time is less than 3 minutes at 37°C ±2°C (without disc), and hardness is from about 30 N to about 70 N.

In one or more embodiments, the dissolution is more than 85% in 15 minutes.

As used herein, the dissolution medium is 900 ml of 0.1N Hydrochloric acid with temperature of 37°C ± 0.5°C and withdrawal time of 15 minutes.

In another embodiment, the present disclosure provides a process for preparation of a lisdexamfetamine dimesylate orodispersible tablet. The process comprises co-sifting lisdexamfetamine dimesylate, a binder, and a diluent; dry mixing the sifted lisdexamfetamine dimesylate, the binder and the diluent; blending the dry mixed lisdexamfetamine dimesylate, the binder and the diluent to prepare a blended dry mix; co-sifting and blending the diluent, a cushioning agent, a disintegrating agent, a sweetener, and an antistatic agent to prepare an intermediate blend; blending the blended dry mix and the intermediate blend; lubricating the blended dry mix and the intermediate blend to form ready to compress drug granules; and compressing the ready to compress drug granules to form compressed lisdexamfetamine dimesylate orodispersible tablet.

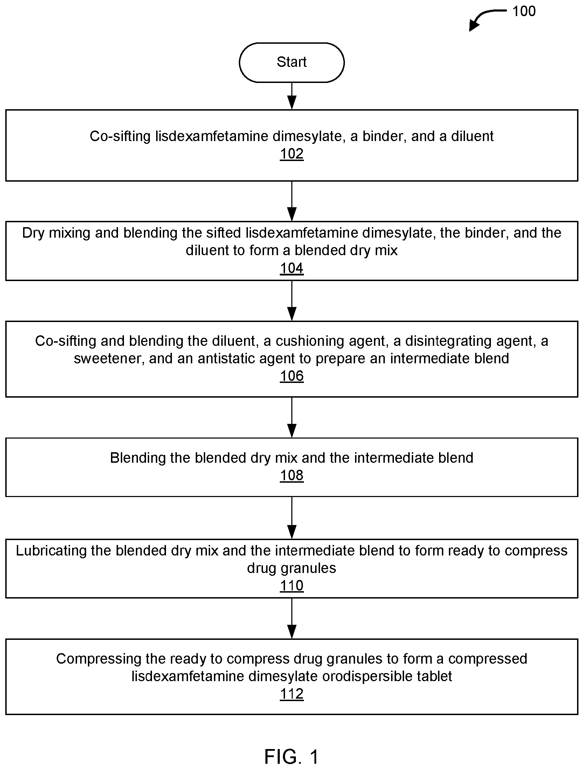

Referring to FIG. 1, the process 100 relates to a process for preparation of lisdexamfetamine dimesylate orodispersible tablet. At step 102, the process 100 comprises co-sifting lisdexamfetamine dimesylate, a binder, and a diluent. At step 104, the process 100 comprises dry mixing and blending the sifted lisdexamfetamine dimesylate, the binder and the diluent to form a blended dry mix. At step 106, the process 100 comprises co-sifting and blending the diluent, a cushioning agent, a disintegrating agent, a sweetener, and an antistatic agent to prepare an intermediate blend. At step 108, the process 100 comprises blending the blended dry mix and the intermediate blend. At step 110, the process 100 comprises lubricating the blended dry mix and the intermediate blend to form ready to compress drug granules. At step 112, the process 100 comprises compressing the ready to compress drug granules to form compressed lisdexamfetamine dimesylate orodispersible tablet. Thereafter, the compressed lisdexamfetamine dimesylate orodispersible tablets are packed in moisture resistant ALU peel off blister package / ALU-ALU package. The moisture resistant ALU peel off blister package /ALU-ALU package contributes to the humidity-controlled manufacturing process and packaging.

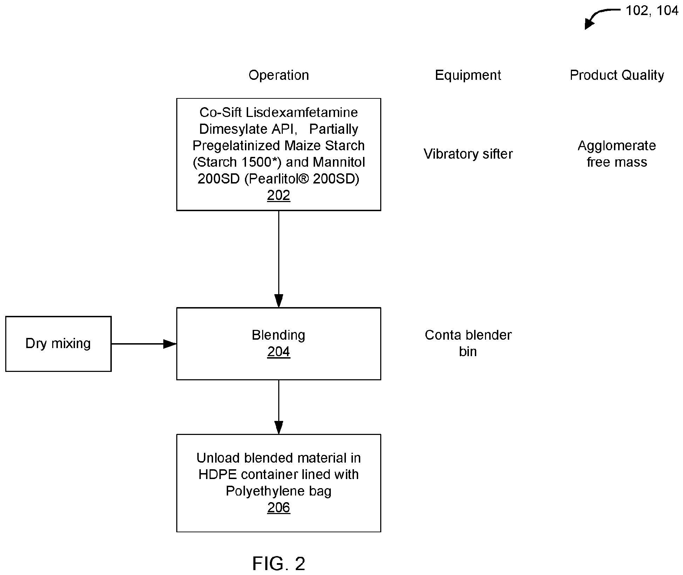

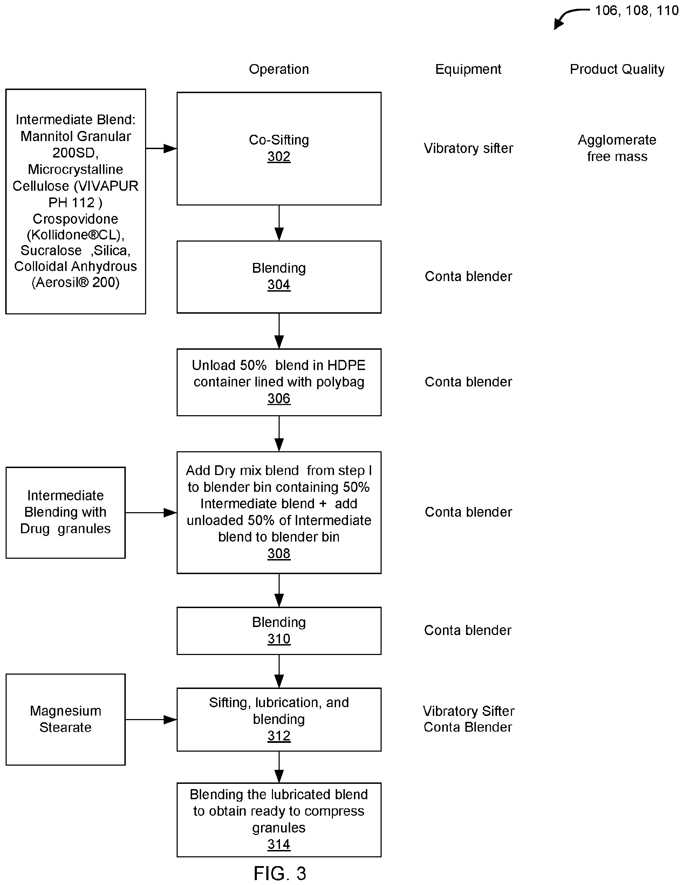

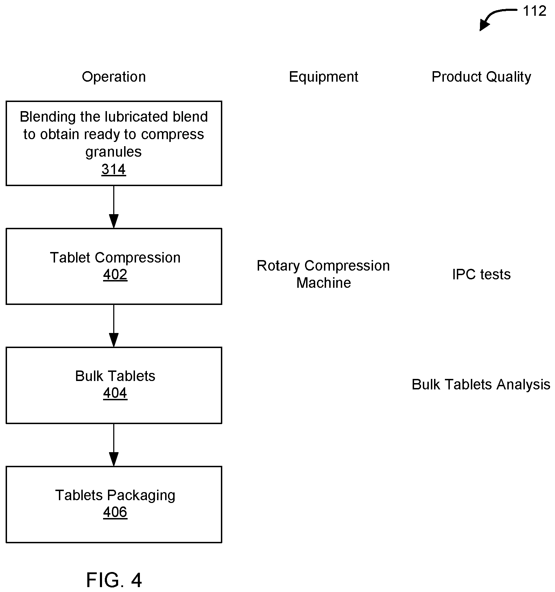

The process steps 102, and 104 of FIG. 1 are described in detail in FIG. 2. The process steps 106, 108, and 110 of FIG. 1 are described in detail in FIG. 3. The process step 112 of FIG. 1 is described in detail in FIG. 4.

Again, referring to FIG. 1, at step 102, the process 100 involves co-sifting lisdexamfetamine dimesylate, a binder, and a diluent, wherein the lisdexamfetamine dimesylate is an active pharmaceutical ingredient (API). The binder is partially pregelatinized maize starch. The diluent is mannitol granular 200SD. At step 104, the process 100 involves dry mixing and blending the agglomerate free mass of the sifted lisdexamfetamine dimesylate partially pregelatinized maize starch and mannitol granular 200SD to form a blended dry mix. The process steps 102 and 104 of FIG. 1 is described in detail in FIG. 2.

Referring to FIG. 2, at step 202, lisdexamfetamine dimesylate, partially pregelatinized maize starch, and mannitol granular 200SD are sifted to obtain agglomerate free mass. The agglomerate free mass of the sifted lisdexamfetamine dimesylate, partially pregelatinized maize starch, and mannitol granular 200SD is collected.

Referring to FIG. 2, at step 204, the agglomerate free mass of the sifted lisdexamfetamine dimesylate, partially pregelatinized maize starch, and mannitol granular 200SD is loaded into a conta blender to dry mix and blend the agglomerate free mass in the conta blender to obtain a blended dry mix. Thereafter, at step 206, the blended dry mix is unloaded.

Again, referring to FIG. 1, at step 106, the next step in the process 100 involves co-sifting and blending the diluent, a cushioning agent, a disintegrating agent, a sweetener, and an antistatic agent to prepare an intermediate blend. The diluent is mannitol granular 200SD. The cushioning agent is microcrystalline cellulose. The disintegrating agent is crospovidone. The sweetener is sucralose. The antistatic agent is colloidal anhydrous silica. The process step 106 of FIG. 1 is described in detail in FIG. 3.

Referring to FIG. 1, at step 108, the next step in the process 100 involves blending the blended dry mix obtained at step 204 and the intermediate blend obtained at step 304. The step 108 of FIG. 1 is described in detail in FIG. 3.

Again, referring to FIG. 1, at step 110, the next step in the process 100 involves lubricating the blended dry mix and the intermediate blend to form ready to compress drug granules. The step 110 of FIG. 1 is described in detail in FIG. 3.

Referring to FIG. 3, at step 302, mannitol granular 200SD, microcrystalline cellulose, crospovidone, sucralose, colloidal anhydrous silica are co-sifted through 36 mesh of the vibratory sifter to obtain agglomerate free mass. At step 304, the sifted material obtained at step 302 is blended in the conta blender at 8 RPM for 30 minutes to obtain the intermediate blend. Thereafter, at step 306, the 50% of intermediated blend obtained at step 304 is unloaded.

Again, referring to FIG. 3, the process step 308 comprises adding blended dry mix obtained at step 204 in FIG. 2 with the 50% of intermediate blend obtained at step 304. Further, adding the 50% intermediate blend unloaded obtained at step 306 to the blender bin containing 50% of the intermediate blend and blended dry mix. Further, at step 310, the above added mixture obtained at step 308 is blended.

Again, referring to FIG. 3, at step 312, the blended mixture obtained at the step 308 is lubricated with a lubricant. Herein, the lubricant is magnesium stearate. The magnesium stearate is sifted through the vibratory sifter and added to the blended mixture in the conta blender to obtain the lubricated blended mixture. The lubricated blended mixture is further blended to obtain ready to compress drug granules at step 314. The ready to compress drug granules are unloaded. Thereafter, the ready to compress drug granules are transferred for compression.

In one or more embodiments, the step of compressing employs direct compression technique in controlled humidity level of NMT 40 % to reduce exposure to moisture.

Again, referring to FIG. 1, at step 112, the process 100 involve compressing the ready to compress drug granules to form compressed lisdexamfetamine dimesylate orodispersible tablet. The step 112 of FIG. 1 is described in detail in FIG. 4. Referring to FIG. 4, at step 402, the ready to compress drug granules obtained at step 314 are compressed through a compression machine to obtain compressed lisdexamfetamine dimesylate orodispersible tablets. The compression used herein is a direct compression technique during the manufacturing process in controlled humidity level of NMT 40 % to reduce exposure to moisture.

After compression of lisdexamfetamine dimesylate orodispersible tablets, at step 404, the bulk compressed tablets are stored in black coloured double-lined polybags in HDPE drums containing silica gel bags in pouch as desiccant. Drums are stored at temperature less than 25°C in the quarantine area before primary packaging. At step 406, the compressed lisdexamfetamine dimesylate orodispersible bulk tablets are packaged. In one embodiment, the compressed lisdexamfetamine dimesylate orodispersible tablets are packed in moisture resistant ALU peel off blister package. In another embodiment, the compressed lisdexamfetamine dimesylate orodispersible tablets are packed in moisture resistant ALU-ALU package.

Herein, the compression yield is more than 95%.

In one or more embodiments, the compressed lisdexamfetamine dimesylate orodispersible tablet is packed in a moisture resistant (ALU) peel off blister package / ALU-ALU package using a blister packing machine. The moisture resistant ALU peel off blister package / ALU-ALU package contributes to the humidity-controlled manufacturing process and packaging.

In TABLE 1, the weight percentage of API and excipients for the exhibit batch is depicted.

**TABLE 1**

| Ingredients | Relative Qty. (% w/w) |
|---|---|
| Lisdexamfetamine dimesylate | 10.0 |
| Mannitol Granular 200SD | 51.75 |
| Partially pregelatinized Maize Starch (Starch1500^{®}) | 15.0 |
| Microcrystalline Cellulose PH112 | 10.0 |
| Crospovidone | 8.0 |
| Sucralose | 2.5 |
| Silica, Colloidal anhydrous | 1.5 |
| Magnesium Stearate | 1.25 |
| Total | 100.0 |

The lisdexamfetamine dimesylate orodispersible tablet and process for preparation thereof is further illustrated by the following non-limiting examples. A person skilled in the art would recognize that the specific examples are intended to illustrate, not limit, the scope of the present disclosure.

In TABLE 2, the physical characteristics (in mg/tablet) for 10, 20, 30, 40, 50, 60, and 70 mg/tablets of lisdexamfetamine dimesylate orodispersible tablet are depicted.

**TABLE 2**

| **Sr. No** | **Parameters** | **Lisdexamfetamine Dimesylate ODT** | | | | | | |
|---|---|---|---|---|---|---|---|---|
| | | **10 mg** | **20 mg** | **30 mg** | **40 mg** | **50 mg** | **60 mg** | **70 mg** |
| **1.** | **Appearance** | White to off white, round, flat beveled edge tablet plain on both surfaces | White to off white, round, flat beveled edge tablet plain on both surfaces | White to off white, round, flat beveled edge tablet plain on both surfaces | White to off white, round, flat beveled edge tablet plain on both surfaces | White to off white, round, flat beveled edge tablet plain on both surfaces | White to off white, round, flat beveled edge tablet plain on both surfaces | White to off white, round, flat beveled edge tablet plain on both surfaces |
| **2.** | **Average Weight of Tablet** | 100.0 mg | 200.0 mg | 300.0 mg | 400.0 mg | 500.0 mg | 600.0 mg | 700.0 mg |
| **3.** | **Weight of 20 Tablets** | 2.0 g | 4.0 g | 6.0 g | 8.0 g | 10.0 g | 12.0 g | 14.0 g |
| **4.** | **Thickness** | 2.2 mm | 3.6 mm | 3.50 mm | 3.80 mm | 4.0 mm | 4.1 mm | 4.0 mm |
| **5.** | **Diameter** | 7 mm | 8 mm | 10 mm | 11 mm | 12 mm | 13 mm | 14 mm |
| **6.** | **Resistance to crushing strength of tablet / Hardness** | 30 N | 35 N | 40 N | 50 N | 50 N | 60 N | 70 N |
| **7.** | **Disintegration time** | NMT 3.0 minutes at 37°C ±2°C (without disc) | | | | | | |
| **8.** | **Friability** | 0.124% | 0.05% | 0.44% | 0.33% | 0.47% | 0.65% | 0.36% |

### EXAMPLE 1

In Example 1, a process for preparing lisdexamfetamine dimesylate orodispersible tablet is described. Particularly, the process is described for preparing lisdexamfetamine dimesylate orodispersible tablet having strength of 10 mg per tablet.

In TABLE 3, the amount of API and excipients (in mg/tablet) for Lisdexamfetamine Dimesylate (10 mg) orodispersible tablets are depicted.

**TABLE 3**

| **DRY MIXING** | |
|---|---|
| Ingredients | Relative Qty. (in mg/tablet) |
| Lisdexamfetamine dimesylate | 10.0 mg |
| Mannitol Granular 200SD (Pearlitol^{®} 200SD) | 25.0 mg |
| Partially pregelatinized Maize Starch (Starch1500^{®}) | 15.0 mg |

| **INTERMEDIATE BLENDING** | |
|---|---|
| Mannitol Granular 200SD (Pearlitol^{®} 200SD) | 26.75 mg |
| Microcrystalline Cellulose (VIVAPUR^{®} 112) | 10.0 mg |
| Crospovidone (Kollidone^{®}CL) | 8.0 mg |
| Sucralose | 2.5 mg |
| Silica, Colloidal anhydrous (Aerosil^{®} 200) | 1.5 mg |

| **LUBRICATION** | |
|---|---|
| Magnesium Stearate | 1.25 mg |
| Total | 100.0mg |

The process is initiated by co-sifting of lisdexamfetamine dimesylate, partially pregelatinized maize starch, and mannitol granular 200SD. Specifically, 10 mg of lisdexamfetamine dimesylate, 25 mg of mannitol granular 200SD, and 15 mg of partially pregelatinized maize starch are sifted through the vibratory sifter to obtain agglomerate free mass. The agglomerate free mass of the sifted lisdexamfetamine dimesylate, partially pregelatinized maize starch, and mannitol granular 200SD is collected.

Thereafter, the agglomerate free mass of the sifted lisdexamfetamine dimesylate, partially pregelatinized maize starch, and mannitol granular 200SD is loaded into a conta blender to dry mix and blend the agglomerate free mass in the conta blender to obtain a blended dry mix and the blended dry mix is unloaded.

Thereafter, 26.75 mg of mannitol granular 200SD, 10 mg of microcrystalline cellulose, 8 mg of crospovidone, 2.5 mg of sucralose, 1.5 mg of colloidal anhydrous silica are co-sifted through the vibratory sifter to obtain agglomerate free mass. Thereafter, the sifted material obtained is blended in the conta blender to obtain the intermediate blend. Thereafter, the 50% of intermediated blend obtained is unloaded.

In the next step, the blended dry mix is added into the blender bin of the conta blender containing 50% of the intermediated blend and the unloaded 50% intermediate blend is added to the blender bin containing 50% of the intermediate blend and blended dry mix to blend the mixture.

Thereafter, 1.25 mg of magnesium stearate is sifted through the vibratory sifter and added to the blended mixture in the conta blender to obtain the lubricated blended mixture. The lubricated blended mixture is further blended to obtain ready to compress drug granules. The ready to compress drug granules are unloaded and transferred for compression.

Finally, the ready to compress drug granules are compressed through a compression machine to obtain a compressed Lisdexamfetamine Dimesylate (10 mg) orodispersible tablet. The compression used herein is a direct compression technique during the manufacturing process in controlled humidity level of NMT 40 % to reduce exposure to moisture.

In TABLE 3A, the product description of the compressed Lisdexamfetamine Dimesylate (10 mg) orodispersible tablet is depicted.

**TABLE 3A**

| **PRODUCT DESCRIPTION** | |
|---|---|
| Tablet Weight | 100 mg ± 7.5 % |
| Hardness | 30 N |
| Thickness | 2.2 mm ±0.3 mm |
| Diameter | 7 mm ±0.3 mm |
| Disintegration Time (DT) | NMT 3 min at 37°C ± 2 °C (without disc) |
| Friability | NMT 2% |

In TABLE 3B, the physical characteristics of the compressed Lisdexamfetamine Dimesylate (10 mg) orodispersible tablet are depicted.

**TABLE 3B**

| **Product Name** | Lisdexamfetamine Dimesylate orodispersible tablet (10 mg) |
|---|---|
| Appearance (Color) | White to off-white |
| Shape | Flat beveled edge round, plain on both surfaces |
| Size (Thickness) | 2.2 mm ±0.3 mm |

### EXAMPLE 2

In Example 2, a process for preparing lisdexamfetamine dimesylate orodispersible tablet is described. Particularly, the process is described for preparing lisdexamfetamine dimesylate orodispersible tablet having strength of 20 mg per tablet.

In TABLE 4, the amount of API and excipients (in mg/tablet) for Lisdexamfetamine Dimesylate (20 mg) orodispersible tablets are depicted.

**TABLE 4**

| **DRY MIXING** | |
|---|---|
| Ingredients | Relative Qty. (in mg/tablet) |
| Lisdexamfetamine dimesylate | 20.0 mg |
| Mannitol Granular 200SD (Pearlitol^{®}200SD) | 50.0 mg |
| Partially pregelatinized Maize Starch (Starch1500^{®}) | 30.0 mg |

| **INTERMEDIATE BLENDING** | |
|---|---|
| Mannitol Granular 200SD (Pearlitol^{®}200SD) | 53.50 mg |
| Microcrystalline Cellulose (VIVAPUR^{®} 112) | 20.0 mg |
| Crospovidone (Kollidone^{®}CL) | 16.0 mg |
| Sucralose | 5.0 mg |
| Silica, Colloidal anhydrous (Aerosil^{®} 200) | 3.0 mg |

| **LUBRICATION** | |
|---|---|
| Magnesium Stearate | 2.5 mg |
| Total | 200.0 mg |

The process is initiated by co-sifting of lisdexamfetamine dimesylate, mannitol granular 200SD, and partially pregelatinized maize starch. Specifically, 20 mg of lisdexamfetamine dimesylate, 50.0 mg of mannitol granular 200SD, and 30.0 mg of partially pregelatinized maize starch are sifted through the vibratory sifter to obtain agglomerate free mass. The agglomerate free mass of the sifted lisdexamfetamine dimesylate, mannitol granular 200SD, and partially pregelatinized maize starch is collected.

Thereafter, the agglomerate free mass of the sifted lisdexamfetamine dimesylate, mannitol granular 200SD, and partially pregelatinized maize starch is loaded into a conta blender to dry mix and blend the agglomerate free mass in the conta blender to obtain a blended dry mix and the blended dry mix is unloaded.

Thereafter, 53.50 mg of mannitol granular 200SD, 20 mg of microcrystalline cellulose, 16 mg of crospovidone, 5 mg of sucralose, 3 mg of colloidal anhydrous silica are co-sifted through the vibratory sifter to obtain agglomerate free mass. Thereafter, the sifted material obtained is blended in the conta blender to obtain the intermediate blend. Thereafter, the 50% of intermediated blend obtained is unloaded.

In the next step, the blended dry mix is added into the blender bin of the conta blender containing 50% of the intermediated blend and the unloaded 50% intermediate blend is added to the blender bin containing 50% of the intermediate blend and blended dry mix to blend the mixture.

Thereafter, 2.5 mg of magnesium stearate is sifted through the vibratory sifter and added to the blended mixture in the conta blender to obtain the lubricated blended mixture. The lubricated blended mixture is further blended to obtain ready to compress drug granules. The ready to compress drug granules are unloaded and transferred for compression.

Finally, the ready to compress drug granules are compressed through a compression machine to obtain a compressed Lisdexamfetamine Dimesylate (20 mg) orodispersible tablet. The compression used is a direct compression technique during the manufacturing process in controlled humidity level of NMT 40 % to reduce exposure to moisture.

In TABLE 4A, the product description of the compressed Lisdexamfetamine Dimesylate (20 mg) orodispersible tablet is depicted.

**TABLE 4A**

| **PRODUCT DESCRIPTION** | |
|---|---|
| Tablet Weight | 200 mg ± 7.5 % |
| Hardness | 35 N |
| Thickness | 3.6 mm ±0.3 mm |
| Diameter | 8 mm ±0.3 mm |
| Disintegration Time (DT) | NMT 3 min at 37°C ± 2 °C (without disc) |
| Friability | NMT 2 % |

In TABLE 4B, the physical characteristics of the compressed Lisdexamfetamine Dimesylate (20 mg) orodispersible tablet are depicted.

**TABLE 4B**

| **Product Name** | Lisdexamfetamine Dimesylate orodispersible tablet (20 mg) |
|---|---|
| Appearance (Color) | White to off-white |
| Shape | Flat beveled edge round, plain on both surfaces |
| Size (Thickness) | 3.6 mm ±0.3 mm |

### EXAMPLE 3

In Example 3, a process for preparing lisdexamfetamine dimesylate orodispersible tablet is described. Particularly, the process is described for preparing lisdexamfetamine dimesylate orodispersible tablet having strength of 30 mg per tablet.

In TABLE 5, the amount of API and excipients (in mg/tablet) for Lisdexamfetamine Dimesylate (30 mg) orodispersible tablets are depicted.

**TABLE 5**

| **DRY MIXING** | |
|---|---|
| Ingredients | Relative Qty. (in mg/tablet) |
| Lisdexamfetamine dimesylate | 30.0 mg |
| Mannitol Granular 200SD (Pearlitol^{®}200SD) | 75.0 mg |
| Partially pregelatinized Maize Starch (Starch1500^{®}) | 45.0 mg |

| **INTERMEDIATE BLENDING** | |
|---|---|
| Mannitol Granular 200SD (Pearlitol^{®} 200SD) | 80.25 mg |
| Microcrystalline Cellulose (VIVAPUR^{®} 112) | 30.0 mg |
| Crospovidone (Kollidone^{®}CL) | 24.0 mg |
| Sucralose | 7.5 mg |
| Silica, Colloidal anhydrous (Aerosil^{®} 200) | 4.5 mg |

| **LUBRICATION** | |
|---|---|
| Magnesium Stearate | 3.75 mg |
| Total | 300.0mg |

The process is initiated by co-sifting of lisdexamfetamine dimesylate, mannitol granular 200SD, and partially pregelatinized maize starch. Specifically, 30 mg of lisdexamfetamine dimesylate, 75 mg of mannitol granular 200SD, and 45 mg of partially pregelatinized maize starch are sifted through the vibratory sifter to obtain agglomerate free mass. The agglomerate free mass of the sifted lisdexamfetamine dimesylate, mannitol granular 200SD, and partially pregelatinized maize starch is collected.

Thereafter, the agglomerate free mass of the sifted lisdexamfetamine dimesylate, mannitol granular 200SD, and partially pregelatinized maize starch is loaded into a conta blender to dry mix and blend the agglomerate free mass in the conta blender to obtain a blended dry mix and the blended dry mix is unloaded.

Thereafter, 80.25 mg of mannitol granular 200SD, 30 mg of microcrystalline cellulose, 24 mg of crospovidone, 7.5 mg of sucralose, 4.5 mg of colloidal anhydrous silica are co-sifted through the vibratory sifter to obtain agglomerate free mass. Thereafter, the sifted material obtained is blended in the conta blender to obtain the intermediate blend. Thereafter, the 50% of intermediated blend obtained is unloaded.

In the next step, the blended dry mix is added into the blender bin of the conta blender containing 50% of the intermediated blend and the unloaded 50% intermediate blend is added to the blender bin containing 50% of the intermediate blend and blended dry mix to blend the mixture.

Thereafter, 3.75 mg of magnesium stearate is sifted through the vibratory sifter and added to the blended mixture in the conta blender to obtain the lubricated blended mixture. The lubricated blended mixture is further blended to obtain ready to compress drug granules. The ready to compress drug granules are unloaded and transferred for compression.

Finally, the ready to compress drug granules are compressed through a compression machine to obtain a compressed Lisdexamfetamine Dimesylate (30 mg) orodispersible tablet. The compression used is a direct compression technique during the manufacturing process in controlled humidity level of NMT 40 % to reduce exposure to moisture.

In TABLE 5A, the amount of API and excipients (in mg/tablet) for Lisdexamfetamine Dimesylate (30 mg) orodispersible tablets are depicted.

**TABLE 5A**

| **PRODUCT DESCRIPTION** | |
|---|---|
| Tablet Weight | 300 mg ± 5 % |
| Hardness | 40 N |
| Thickness | 3.50 mm ±0.3 mm |
| Diameter | 10 mm ±0.3 mm |
| Disintegration Time (DT) | NMT 3 min at 37°C ± 2 °C (without disc) |
| Friability | NMT 2 % |

In TABLE 5B, the physical characteristics of the compressed Lisdexamfetamine Dimesylate (30 mg) orodispersible tablet are depicted.

**TABLE 5B**

| **Product Name** | Lisdexamfetamine Dimesylate orodispersible tablet (30 mg) |
|---|---|
| Appearance (Color) | White to off-white |
| Shape | Flat beveled edge round, plain on both surfaces |
| Size (Thickness) | 3.50 mm ±0.3 mm |

### EXAMPLE 4

In example 4, a process for preparing lisdexamfetamine dimesylate orodispersible tablet is described. Particularly, the process is described for preparing lisdexamfetamine dimesylate orodispersible tablet having strength of 40 mg per tablet.

In TABLE 6, the amount of API and excipients (in mg/tablet) for Lisdexamfetamine Dimesylate (40 mg) orodispersible tablets are depicted.

**TABLE 6**

| **DRY MIXING** | |
|---|---|
| Ingredients | Relative Qty. (in mg/tablet) |
| Lisdexamfetamine dimesylate | 40.0 mg |
| Mannitol Granular 200SD (Pearlitol^{®}200SD) | 100.0 mg |
| Partially pregelatinized Maize Starch (Starch1500^{®}) | 60.0 mg |

| **INTERMEDIATE BLENDING** | |
|---|---|
| Mannitol Granular 200SD (Pearlitol^{®} 200SD) | 107.0 mg |
| Microcrystalline Cellulose (VIVAPUR^{®} 112) | 40.0 mg |
| Crospovidone (Kollidone^{®}CL) | 32.0 mg |
| Sucralose | 10.0 mg |
| Silica, Colloidal anhydrous (Aerosil^{®} 200) | 6.0 mg |

| **LUBRICATION** | |
|---|---|
| Magnesium Stearate | 5.0 mg |
| Total | 400.0mg |

The process is initiated by co-sifting of lisdexamfetamine dimesylate, mannitol granular 200SD, and partially pregelatinized maize starch. Specifically, 40 mg of lisdexamfetamine dimesylate, 100 mg of mannitol granular 200SD, and 60 mg of partially pregelatinized maize starch are sifted through the vibratory sifter to obtain agglomerate free mass. The agglomerate free mass of the sifted lisdexamfetamine dimesylate, mannitol granular 200SD, and partially pregelatinized maize starch is collected.

Thereafter, the agglomerate free mass of the sifted lisdexamfetamine dimesylate, mannitol granular 200SD, and partially pregelatinized maize starch is loaded into a conta blender to dry mix and blend the agglomerate free mass in the conta blender to obtain a blended dry mix and the blended dry mix is unloaded.

Thereafter, 107 mg of mannitol granular 200SD, 40 mg of microcrystalline cellulose, 32 mg of crospovidone, 10 mg of sucralose, and 6 mg of colloidal anhydrous silica are co-sifted through the vibratory sifter to obtain agglomerate free mass. Thereafter, the sifted material obtained is blended in the conta blender to obtain the intermediate blend. Thereafter, the 50% of intermediated blend obtained is unloaded.

In the next step, the blended dry mix is added into the blender bin of the conta blender containing 50% of the intermediated blend and the unloaded 50% intermediate blend is added to the blender bin containing 50% of the intermediate blend and blended dry mix to blend the mixture.

Thereafter, 5 mg of magnesium stearate is sifted through the vibratory sifter and added to the blended mixture in the conta blender to obtain the lubricated blended mixture. The lubricated blended mixture is further blended to obtain ready to compress drug granules. The ready to compress drug granules are unloaded and transferred for compression.

Finally, the ready to compress drug granules are compressed through a compression machine to obtain a compressed Lisdexamfetamine Dimesylate (40 mg) orodispersible tablet. The compression used is a direct compression technique during the manufacturing process in controlled humidity level of NMT 40 % to reduce exposure to moisture.

In TABLE 6A, the product description of the compressed Lisdexamfetamine Dimesylate (40 mg) orodispersible tablet is depicted.

**TABLE 6A**

| **PRODUCT DESCRIPTION** | |
|---|---|
| Tablet Weight | 400 mg ± 5 % |
| Hardness | 50 N |
| Thickness | 3.80 mm ±0.3 mm |
| Diameter | 11 mm ±0.3 mm |
| Disintegration Time (DT) | NMT 3 min at 37°C ± 2 °C (without disc) |
| Friability | NMT 2 % |

In TABLE 6B, the physical characteristics of the compressed Lisdexamfetamine Dimesylate (40 mg) orodispersible tablet are depicted.

**TABLE 6B**

| **Product Name** | lisdexamfetamine dimesylate orodispersible tablet (40 mg) |
|---|---|
| Appearance (Color) | White to off-white |
| Shape | Flat beveled edge round, plain on both surfaces |
| Size (Thickness) | 3.80 mm ±0.3 mm |

### EXAMPLE 5

In Example 5, a process for preparing lisdexamfetamine dimesylate orodispersible tablet is described. Particularly, the process is described for preparing lisdexamfetamine dimesylate orodispersible tablet having strength of 50 mg per tablet.

In TABLE 7, the amount of API and excipients (in mg/tablet) for Lisdexamfetamine Dimesylate (50 mg) orodispersible tablets are depicted.

**TABLE 7**

| **DRY MIXING** | |
|---|---|
| Ingredients | Relative Qty. (in mg/tablet) |
| Lisdexamfetamine dimesylate | 50.0 mg |
| Mannitol Granular 200SD (Pearlitol^{®}200SD) | 125.0 mg |
| Partially pregelatinized Maize Starch (Starch1500^{®}) | 75.0 mg |

| **INTERMEDIATE BLENDING** | |
|---|---|
| Mannitol Granular 200SD (Pearlitol^{®} 200SD) | 133.75 mg |
| Microcrystalline Cellulose (VIVAPUR^{®} 112) | 50.0 mg |
| Crospovidone (Kollidone^{®}CL) | 40.0 mg |
| Sucralose | 12.5 mg |
| Silica, Colloidal anhydrous (Aerosil^{®} 200) | 7.5 mg |

| **LUBRICATION** | |
|---|---|
| Magnesium Stearate | 6.25 mg |
| Total | 500.0mg |

The process is initiated by co-sifting of lisdexamfetamine dimesylate, mannitol granular 200SD, and partially pregelatinized maize starch. Specifically, 50 mg of lisdexamfetamine dimesylate, 125 mg of mannitol granular 200SD, and 75 mg of partially pregelatinized maize starch are sifted through the vibratory sifter to obtain agglomerate free mass. The agglomerate free mass of the sifted lisdexamfetamine dimesylate, mannitol granular 200SD, and partially pregelatinized maize starch is collected.

Thereafter, the agglomerate free mass of the sifted lisdexamfetamine dimesylate, mannitol granular 200SD, and partially pregelatinized maize starch is loaded into a conta blender to dry mix and blend the agglomerate free mass in the conta blender to obtain a blended dry mix and the blended dry mix is unloaded.

Thereafter, 133.75 mg of mannitol granular 200SD, 50 mg microcrystalline of cellulose, 40 mg of crospovidone, 12.5 mg of sucralose, 7.5 mg of colloidal anhydrous silica are co-sifted through the vibratory sifter to obtain agglomerate free mass. Thereafter, the sifted material obtained is blended in the conta blender to obtain the intermediate blend. Thereafter, the 50% of intermediated blend obtained is unloaded.

In the next step, the blended dry mix is added into the blender bin of the conta blender containing 50% of the intermediated blend and the unloaded 50% intermediate blend is added to the blender bin containing 50% of the intermediate blend and blended dry mix to blend the mixture.

Thereafter, 6.25 mg of magnesium stearate is sifted through of vibratory sifter and added to the blended mixture in the conta blender to obtain the lubricated blended mixture. The lubricated blended mixture is further blended at to obtain ready to compress drug granules. The ready to compress drug granules are unloaded and transferred for compression.

Finally, the ready to compress drug granules are compressed through a compression machine to obtain a compressed Lisdexamfetamine Dimesylate (50 mg) orodispersible tablet. The compression used is a direct compression technique during the manufacturing process in controlled humidity level of NMT 40 % to reduce exposure to moisture.

In TABLE 7A, the product description of the compressed Lisdexamfetamine Dimesylate (50 mg) orodispersible tablet is depicted.

**TABLE 7A**

| **PRODUCT DESCRIPTION** | |
|---|---|
| Tablet Weight | 500 mg ± 5 % |
| Hardness | 50 N |
| Thickness | 4.0 mm ±0.3 mm |
| Diameter | 12 mm ±0.3 mm |
| Disintegration Time (DT) | NMT 3 min at 37°C ± 2 °C (without disc) |
| Friability | NMT 2% |

In TABLE 7B, the physical characteristics of the compressed Lisdexamfetamine Dimesylate (50 mg) orodispersible tablet are depicted.

**TABLE 7B**

| **Product Name** | Lisdexamfetamine Dimesylate orodispersible tablet (50 mg) |
|---|---|
| Appearance (Color) | White to off-white |
| Shape | Flat beveled edge round, plain on both surfaces |
| Size (Thickness) | 4.0 mm ±0.3 mm |

### EXAMPLE 6

In Example 6, a process for preparing lisdexamfetamine dimesylate orodispersible tablet is described. Particularly, the process is described for preparing lisdexamfetamine dimesylate orodispersible tablet having strength of 60 mg per tablet.

In TABLE 8, the amount of API and excipients (in mg/tablet) for Lisdexamfetamine Dimesylate (60 mg) orodispersible tablets are depicted.

**TABLE 8**

| **DRY MIXING** | |
|---|---|
| Ingredients | Relative Qty. (in mg/tablet) |
| Lisdexamfetamine dimesylate | 60.0 mg |
| Mannitol Granular 200SD (Pearlitol^{®}200SD) | 150.0 mg |
| Partially pregelatinized Maize Starch (Starch1500^{®}) | 90.0 mg |

| **INTERMEDIATE BLENDING** | |
|---|---|
| Mannitol Granular 200SD (Pearlitol^{®} 200SD) | 160.5 mg |
| | |
| Microcrystalline Cellulose (VIVAPUR^{®} 112) | 60.0 mg |
| Crospovidone (Kollidone^{®}CL) | 48.0 mg |
| Sucralose | 15.0 mg |
| Silica, Colloidal anhydrous (Aerosil^{®} 200) | 9.0 mg |

| **LUBRICATION** | |
|---|---|
| Magnesium Stearate | 7.5 mg |
| Total | 600.0mg |

The process is initiated by co-sifting of lisdexamfetamine dimesylate, mannitol granular 200SD, and partially pregelatinized maize starch. Specifically, 60 mg of lisdexamfetamine dimesylate, 150 mg of mannitol granular 200SD, and 90 mg of partially pregelatinized maize starch are sifted through the vibratory sifter to obtain agglomerate free mass. The agglomerate free mass of the sifted lisdexamfetamine dimesylate, mannitol granular 200SD, and partially pregelatinized maize starch is collected.

Thereafter, the agglomerate free mass of the sifted lisdexamfetamine dimesylate, mannitol granular 200SD, and partially pregelatinized maize starch is loaded into a conta blender to dry mix and blend the agglomerate free mass in the conta blender to obtain a blended dry mix and the blended dry mix is unloaded.

Thereafter, 160.5 mg of mannitol granular 200SD, 60 mg of microcrystalline cellulose, 48 mg of crospovidone, 15 mg of sucralose, 9 mg of colloidal anhydrous silica are co-sifted through the vibratory sifter to obtain agglomerate free mass. Thereafter, the sifted material obtained is blended in the conta blender to obtain the intermediate blend. Thereafter, the 50% of intermediated blend obtained.

In the next step, the blended dry mix is added into the blender bin of the conta blender containing 50% of the intermediated blend and the unloaded 50% intermediate blend is added to the blender bin containing 50% of the intermediate blend and blended dry mix to blend the mixture.

Thereafter, 7.5 mg of magnesium stearate is sifted through the vibratory sifter and added to the blended mixture in the conta blender to obtain the lubricated blended mixture. The lubricated blended mixture is further blended to obtain ready to compress drug granules. The ready to compress drug granules are unloaded and transferred for compression.

Finally, the ready to compress drug granules are compressed through a compression machine to obtain a compressed Lisdexamfetamine Dimesylate (60 mg) orodispersible tablet. The compression used is a direct compression technique during the manufacturing process in controlled humidity level of NMT 40 % to reduce exposure to moisture.

In TABLE 8A, the product description of the compressed Lisdexamfetamine Dimesylate (60 mg) orodispersible tablet is depicted.

**TABLE 8A**

| **PRODUCT DESCRIPTION** | |
|---|---|
| Tablet Weight | 600 mg ± 5 % |
| Hardness | 60 N |
| Thickness | 4.1 mm ±0.3 mm |
| Diameter | 13 mm ±0.3 mm |
| Disintegration Time (DT) | NMT 3 min at 37°C ± 2 °C (without disc) |
| Friability | NMT 2 % |

In TABLE 8B, the physical characteristics of the compressed Lisdexamfetamine Dimesylate (60 mg) orodispersible tablet are depicted.

**TABLE 8B**

| **Product Name** | Lisdexamfetamine Dimesylate orodispersible tablet (60 mg) |
|---|---|
| Appearance (Color) | White to off-white |
| Shape | Flat beveled edge round, plain on both surfaces |
| Size (Thickness) | 4.1 mm ±0.3 mm |

### EXAMPLE 7

In Example 7, a process for preparing lisdexamfetamine dimesylate orodispersible tablet is described. Particularly, the process is described for preparing lisdexamfetamine dimesylate orodispersible tablet having strength of 70 mg per tablet.

In TABLE 9, the amount of API and excipients (in mg/tablet) for Lisdexamfetamine Dimesylate (70 mg) orodispersible tablets are depicted.

**TABLE 9**

| **DRY MIXING** | |
|---|---|
| Ingredients | Relative Qty. (in mg/tablet) |
| Lisdexamfetamine dimesylate | 70.0 mg |
| Mannitol Granular 200SD (Pearlitol^{®}200SD) | 175.0 mg |
| Partially pregelatinized Maize Starch (Starch1500^{®}) | 105.0 mg |

| **INTERMEDIATE BLENDING** | |
|---|---|
| Mannitol Granular 200SD (Pearlitol^{®}200SD) | 187.25 mg |
| Microcrystalline Cellulose (VIVAPUR^{®} 112) | 70.0 mg |
| Crospovidone (Kollidone^{®}CL) | 56.0 mg |
| | |
| Sucralose | 17.5 mg |
| Silica, Colloidal anhydrous (Aerosil^{®} 200) | 10.5 mg |

| **LUBRICATION** | |
|---|---|
| Magnesium Stearate | 8.75 mg |
| Total | 700.0mg |

The process is initiated by co-sifting of lisdexamfetamine dimesylate, mannitol granular 200SD, and partially pregelatinized maize starch. Specifically, 70 mg lisdexamfetamine dimesylate, 175 mg of mannitol granular 200SD, and 105 mg of partially pregelatinized maize starch are sifted through the vibratory sifter to obtain agglomerate free mass. The agglomerate free mass of the sifted lisdexamfetamine dimesylate, mannitol granular 200SD, and partially pregelatinized maize starch is collected.

Thereafter, the agglomerate free mass of the sifted lisdexamfetamine dimesylate, mannitol granular 200SD, and partially pregelatinized maize starch is loaded into a conta blender to dry mix and blend the agglomerate free mass in the conta blender to obtain a blended dry mix and the blended dry mix is unloaded.

Thereafter, 187.25 mg of mannitol granular 200SD, 70 mg of microcrystalline cellulose, 56 mg of crospovidone, 17.5 mg of sucralose, 10.5 mg of colloidal anhydrous silica are co-sifted through the vibratory sifter to obtain agglomerate free mass. Thereafter, the sifted material obtained is blended in the conta blender to obtain the intermediate blend. Thereafter, the 50% of intermediated blend obtained is unloaded.

In the next step, the blended dry mix is added into the blender bin of the conta blender containing 50% of the intermediated blend and the unloaded 50% intermediate blend is added to the blender bin containing 50% of the intermediate blend and blended dry mix to blend the mixture.

Thereafter, 8.75 mg of magnesium stearate is sifted through the vibratory sifter and added to the blended mixture in the conta blender to obtain the lubricated blended mixture. The lubricated blended mixture is further blended to obtain ready to compress drug granules. The ready to compress drug granules are unloaded and transferred for compression.

Finally, the ready to compress drug granules are compressed through a compression machine to obtain a compressed lisdexamfetamine dimesylate (70 mg) orodispersible tablet. The compression used is a direct compression technique during the manufacturing process in controlled humidity level of NMT 40 % to reduce exposure to moisture.

In TABLE 9A, the product description of the compressed Lisdexamfetamine Dimesylate (70 mg) orodispersible tablet is depicted.

**TABLE 9A**

| **PRODUCT DESCRIPTION** | |
|---|---|
| Tablet Weight | 700 mg ± 5 % |
| Hardness | 70 N |
| Thickness | 4.0 mm ±0.3 mm |
| Diameter | 14 mm ±0.3 mm |
| Disintegration Time (DT) | NMT 3 min at 37°C ± 2 °C (without disc) |
| | NMT 2 % |

In TABLE 9B, the physical characteristics of the compressed Lisdexamfetamine Dimesylate (70 mg) orodispersible tablet are depicted.

**TABLE 9B**

| **Product Name** | lisdexamfetamine dimesylate orodispersible tablet (70 mg) |
|---|---|
| Appearance (Color) | White to off-white |
| Shape | Flat beveled edge round, plain on both surfaces |
| Size (Thickness) | 4.0 mm ±0.3 mm |

The present disclosure provides a lisdexamfetamine dimesylate orodispersible composition which is in the form of lisdexamfetamine dimesylate orodispersible tablet and process for preparation thereof. The lisdexamfetamine dimesylate orodispersible tablet developed by the inventor is an orally disintegrating tablet that permits rapid release of the drug from the granules and allows rapid absorption in the body after administration. Further, the present disclosure provides a lisdexamfetamine dimesylate orodispersible tablet that is stable, palatable, and disintegrates fast. The present disclosure further provides a manufacturing process in which the process time for preparing lisdexamfetamine dimesylate orodispersible composition is low, and the production cost is low. Furthermore, the present disclosure further provides a manufacturing process with controlled humidity to minimize exposure of the deliquescent material to moisture during the production process.

### Tests:

The included data for the test results shows readings with regard to moisture-controlled tablet composition, i.e., tablet in which the deliquescence of API is taken care of, in other words it shows data on how the exact combination of drug along with all different excipients lead to a formulation/composition with no or insignificant deliquescence.

In order to demonstrate the composition comprising lisdexamfetamine dimesylate does not exhibit deliquescent properties, a series of tests have been performed aimed at assessing the stability, durability, and resistance to various forms of degradation of the tablet composition comprising lisdexamfetamine dimesylate.

The tests are accelerated aging test to assess its resistance to degradation over time; chemical resistance test / compatibility test to assess suitability of excipients materials with the lisdexamfetamine dimesylate tablets; moisture absorption test to show no significant increase in absorption of moisture level by the tablet comprising lisdexamfetamine dimesylate.

### Accelerated Aging Test

The tablet composition was subjected to accelerated stability condition, such as elevated temperature of 40 degree C and humidity of 75 % RH exposure to simulate long-term environmental exposure. The performance of the tablet comprising lisdexamfetamine dimesylate is evaluated before and after aging to assess its resistance to degradation over time.

In TABLE 10, the results for the accelerated aging test performed on the tablet composition comprising lisdexamfetamine dimesylate is depicted.

**TABLE 10**

| **Category / Properties** | **Analysis** |
|---|---|
| Initial Hardness | Ranges from 30 N for lower strength (10 mg) and 70 N for higher strength (70 mg) |
| Hardness on keeping the tablets under room temperature for 3 months | Ranges from 30 N for lower strength (10 mg) and 70 N for higher strength (70 mg) |

The result reveals no change in the hardness of the tablet composition comprising lisdexamfetamine dimesylate. No drop or change in the hardness of the tablet confirms that the material's mechanical properties and its ability to withstand stress and deformation without exhibiting signs of deliquescence.

### Chemical Resistance Test / Compatibility Test

The tablet composition was exposed to a range of chemical substances commonly used in ODT formulation to assess the tablet's resistance to chemical attack, including changes in appearance, physical properties, or structural integrity.

For conducting the compatibility study, the tablets are exposed at elevated temperature of 40 degree C and humidity of 75 % RH exposure in open and closed conditions and at temperature of 25 degree C and humidity of 75 % RH exposure in open and closed conditions.

The result reveals positive interpretations indicating a high level of compatibility between the tablet composition comprising lisdexamfetamine dimesylate and chemical substances commonly used in ODT formulation. The positive results from the compatibility study provide strong evidence supporting the suitability of excipients materials for their intended application.

### Moisture Absorption Test

In order to determine the ability of the tablet composition comprising lisdexamfetamine dimesylate to resist moisture absorption by subjecting it to controlled humidity or water immersion tests. The amount of moisture absorbed by the tablet is measured and its impact on dimensional stability, strength, and other relevant properties are assessed.

The tablet composition comprising lisdexamfetamine dimesylate by using KF analyser at different storage conditions temperature of 40 °C / Relative humidity 75%, temperature of 30°C / Relative humidity 75% temperature of 30 °C / Relative humidity 65% and temperature of 25 °C / Relative humidity 60%.

The results reveal that the absorption of moisture content of tablet composition comprising lisdexamfetamine dimesylate remain the same. Summarily, no significant increase in absorption of moisture level is shown by the tablet.

In TABLE 11, the results for Moisture Absorption Test of tablet composition comprising lisdexamfetamine dimesylate for BATCH 1 is depicted.

**TABLE 11**

| **Parameters** | **Initial** | **1 month** | | | |
|---|---|---|---|---|---|
| | | **25°C/ 60%RH** | **30°C/ 65%RH** | **30°C/ 75%RH** | **40°C/ 75%RH** |
| **Average mass** | 702.2 mg | 700.8 mg | 704.1 mg | 704.6 mg | 703.6 mg |
| **Water Content** | 3.3% | 3.8% | 3.5% | 3.6% | 3.9% |
| **Disintegration Time** | 25 secs | 25 secs | 27 secs | 26 secs | 23 secs |
| **Dissolution (Paddle, 50 rpm, 900 ml 0.1N HCL)** | 100% | 101% | 100% | 102% | 101% |
| **Assay (By HPLC)** | 71.15 mg / tablet | 68.80 mg / tablet | 70.69 mg / tablet | 70.08 mg / tablet | 69.71 mg / tablet |
| | 101.6% | 98.3% | 101.0% | 100.1% | 99.6% |

In TABLE 12, the results for Moisture Absorption Test of tablet composition comprising lisdexamfetamine dimesylate for BATCH 2 is depicted.

**TABLE 12**

| **Parameters** | **Initial** | **1 month** | | | |
|---|---|---|---|---|---|
| | | **25°C/ 60%RH** | **30°C/ 65%RH** | **30°C/ 75%RH** | **40°C/ 75%RH** |
| **Average mass** | 703.0 mg | 703.6 mg | 702.1 mg | 705.3 mg | 703.6 mg |
| **Water Content** | 3.4% | 3.8% | 3.4% | 3.9% | 3.4% |
| **Disintegration Time** | 23 secs | 22 secs | 23 secs | 31 secs | 28 secs |
| **Dissolution (Paddle, 50 rpm, 900 ml 0.1N HCL)** | 97% | 101% | 102% | 96% | 101% |
| **Assay (By HPLC)** | 70.87 mg / tablet | 69.64 mg / tablet | 69.62 mg / tablet | 70.32 mg / tablet | 69.05 mg / tablet |
| | 101.2 % | 99.5% | 99.5% | 100.5% | 98.6% |

In TABLE 13, the results for Moisture Absorption Test of tablet composition comprising lisdexamfetamine dimesylate for BATCH 3 is depicted.

**TABLE 13**

| **Parameters** | **Initial** | **1 month** | | | |
|---|---|---|---|---|---|
| | | **25°C/ 60%RH** | **30°C/ 65%RH** | **30°C/ 75%RH** | **40°C/ 75%RH** |
| **Average mass** | 703.3 mg | 704.0 mg | 703.2 mg | 702.3 mg | 700.8 mg |
| **Water Content** | 3.1% | 3.6% | 3.4% | 3.6% | 3.3% |
| **Disintegration Time** | 24 secs | 26 secs | 27 secs | 28 secs | 25 secs |
| **Dissolution** | | | | | |
| **(Paddle, 50 rpm, 900 ml 0.1N HCL)** | 98% | 99% | 102% | 102% | 96% |
| **Assay (By HPLC)** | 70.71 mg / tablet | 69.40 mg / tablet | 70.66 mg / tablet | 70.06 mg / tablet | 69.20 mg / tablet |
| | 101.0% | 99.1 % | 100.9% | 100.1% | 98.9% |

The stability report for BATCH 1, BATCH 2, and BATCH 3 emphasizes on non-deliquescent nature of the lisdexamfetamine dimesylate orodispersible composition. From the stability data (shown in TABLE 11, TABLE 12 and TABLE 11), it can be interpreted that there is no significant increase in water content at mentioned storage conditions. Hence, in other words it can be concluded that lisdexamfetamine dimesylate composition for orodispersible tablet is stable.

The present disclosure provides a synergistic combination of the API with the excipients in specific ratio to address the delinquent nature of lisdexamfetamine dimesylate. The synergistic combination enhances the disintegration time of the orodispersible tablet. The present disclosure further imparts improved palatability to the tablet formulation of lisdexamfetamine dimesylate. The present disclosure provides a humidity-controlled manufacturing process wherein the tablet formulation is manufactured in an environment with controlled humidity to minimize exposure of the deliquescent material to moisture during the production process. Particularly, the manufacturing process employs direct compression techniques during the manufacturing process in controlled humidity level of NMT 40 % to reduce exposure to moisture.

The present disclosure provides a lisdexamfetamine dimesylate composition of orally disintegrating tablet which overcomes the drawbacks of capsules and chewable tablets. The synergistic combination of the API with the excipients in specific ratio is formulated to address the deliquescent nature of lisdexamfetamine dimesylate and provide a stable lisdexamfetamine dimesylate composition. The dissolution of the lisdexamfetamine orodispersible tablet composition is more than 85% in 15 minutes.

The foregoing descriptions of specific embodiments of the present disclosure have been presented for purposes of illustration and description. They are not intended to be exhaustive or to limit the present disclosure to the precise forms disclosed, and obviously many modifications and variations are possible in light of the above teaching. The embodiments were chosen and described in order to best explain the principles of the present disclosure and its practical application, to thereby enable others skilled in the art to best utilize the present disclosure and various embodiments with various modifications as are suited to the particular use contemplated. (It is understood that various omissions and substitutions of equivalents are contemplated as circumstance may suggest or render expedient, but such are intended to cover the application or implementation without departing from the spirit or scope of the claims of the present disclosure).

## Claims

1. A lisdexamfetamine dimesylate orodispersible tablet, the lisdexamfetamine dimesylate orodispersible tablet comprising:
about 8 to about 12 percent by weight of lisdexamfetamine dimesylate;
about 45 to about 55 percent by weight of a diluent;
about 10 to about 20 percent by weight of a binder;
about 8 to about 12 percent by weight of a cushioning agent;
about 6 to about 10 percent by weight of a disintegrating agent;
about 1 to about 4 percent by weight of a sweetener;
about 1 to about 2 percent by weight of an antistatic agent; and
about 1 to about 2 percent by weight of a lubricant.

2. The lisdexamfetamine dimesylate orodispersible tablet as claimed in claim 1, wherein the dosage amount of the lisdexamfetamine dimesylate orodispersible tablet is 10 mg per tablet; 20 mg per tablet; 30 mg per tablet; 40 mg per tablet; 50 mg per tablet; 60 mg per tablet; and 70 mg per tablet.

3. The lisdexamfetamine dimesylate orodispersible tablet as claimed in claim 1, wherein the diluent is mannitol granular 200SD and the binder is partially pregelatinized maize starch.

4. The lisdexamfetamine dimesylate orodispersible tablet as claimed in claim 1, wherein the cushioning agent is microcrystalline cellulose, the disintegrating agent is crospovidone, the sweetener is sucralose, the antistatic agent is colloidal anhydrous silica, and lubricant is magnesium stearate.

5. The lisdexamfetamine dimesylate orodispersible tablet as claimed in claim 1, wherein the particle size for a drug granule forming the lisdexamfetamine dimesylate orodispersible tablet is not more than 400 microns.

6. The lisdexamfetamine dimesylate orodispersible tablet as claimed in claim 1, wherein the friability is less than 2%, the disintegration time is less than 3 minutes at 37°C ±2°C (without disc), and the hardness is from about 30 N to about 70 N.

7. The lisdexamfetamine dimesylate orodispersible tablet as claimed in claim 1, wherein the dissolution is more than 85% in 15 minutes.

8. The lisdexamfetamine dimesylate orodispersible tablet as claimed in claim 1, wherein the particle size of the lisdexamfetamine dimesylate is not more than 100 microns.

9. A process for preparation of a lisdexamfetamine dimesylate orodispersible tablet, the process comprising:
co-sifting lisdexamfetamine dimesylate, a binder, and a diluent;
dry mixing and blending the sifted lisdexamfetamine dimesylate, the binder and the diluent to form a blended dry mix;
co-sifting and blending the diluent, a cushioning agent, a disintegrating agent, a sweetener, and an antistatic agent to prepare an intermediate blend;
blending the blended dry mix and the intermediate blend;
lubricating the blended dry mix and the intermediate blend to form ready to compress drug granules; and
compressing the ready to compress drug granules to form compressed lisdexamfetamine dimesylate orodispersible tablet.

10. The process as claimed in claim 9, wherein the dosage amount of the lisdexamfetamine dimesylate orodispersible tablet is 10 mg per tablet; 20 mg per tablet; 30 mg per tablet; 40 mg per tablet; 50 mg per tablet; 60 mg per tablet; and 70 mg per tablet.

11. The process as claimed in claim 9, wherein the diluent is mannitol granular 200SD and the binder is partially pregelatinized maize starch.

12. The process as claimed in claim 9, wherein the cushioning agent is microcrystalline cellulose, the disintegrating agent is crospovidone, the sweetener is sucralose, the antistatic agent is colloidal anhydrous silica, and the lubricant is magnesium stearate.

13. The process as claimed in claim 9, wherein the friability is less than 2%, the disintegration time is less than 3 minutes at 37°C ±2°C (without disc), and the hardness is from about 30 N to about 70 N.

14. The process as claimed in claim 9, wherein the step of compressing employs direct compression technique in controlled humidity level of NMT 40 % to reduce exposure to moisture.

15. The process as claimed in claim 9, further comprising packaging of compressed lisdexamfetamine dimesylate orodispersible tablets in moisture resistant ALU peel off blister package.

16. The process as claimed in claim 9, further comprising packaging of compressed lisdexamfetamine dimesylate orodispersible tablets in moisture resistant ALU-ALU package.
